# EUROPÄISCHE PATENTSCHRIFT

(11) **EP 1 506 159 B1**
(45) Veröffentlichungstag und Bekanntmachung des Hinweises auf die Patenterteilung: **19.09.2007**
(21) Anmeldenummer: 03720564.8
(22) Anmeldetag: 12.05.2003
(51) Int. Cl.: C07C 227/38, C07C 229/52

(54) **VERFAHREN ZUR HERSTELLUNG VON 2-(4-N, N-DIALKYLAMINO-2-HYDROXYBENZOYL)BENZOES UREESTERN**
METHOD FOR PRODUCING 2-(4-N, N-DIALKYLAMINO-2-HYDROXYBENZOYL)BENZOATES
PROCEDE DE PRODUCTION DE BENZOATE DE 2-(4-N,N-DIALKYLAMINO-2-HYDROXYBENZOYLE)

(30) Priorität: 15.05.2002 DE 10221805
(43) Veröffentlichungstag der Anmeldung: 16.02.2005
(73) Patentinhaber: BASF Aktiengesellschaft, 67056 Ludwigshafen (DE)
(72) Erfinder: HEIDENFELDER, Thomas, 67125 Dannstadt-Schauernheim (DE); BECK, Karl, 76684 Östringen (DE)
(86) Internationale Anmeldenummer: PCT/EP2003/004918
(87) Internationale Veröffentlichungsnummer: WO 2003/097578

(56) Entgegenhaltungen:
- EP-A- 1 046 391
- PATENT ABSTRACTS OF JAPAN vol. 010, no. 350 (C-387), 26. November 1986 (1986-11-26) & JP 61 151158 A (TAOKA CHEM CO LTD), 9. Juli 1986 (1986-07-09)

## Beschreibung

Die Erfindung betrifft ein Verfahren zur Herstellung von 2-(4-N,N-Diethylamino-2-hydroxybenzoyl)benzoesäure-n-hexylester.

Sonnenlicht, das an die Erdoberfläche gelangt, hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Dermatologische Untersuchungen haben gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A- und UV-B-Bereich notwendig erscheinen.

Substanzen, die eine Benzophenonstruktur 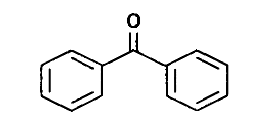 besitzen, zeichnen sich durch sehr gute Absorptionseigenschaften im UV-A-Bereich aus. Vertreter dieser Stoffklasse sind u.a. 2-(4-N,N-Dialkylamino-2-hydroxybenzoyl)benzoesäurealkylester. Deren Verwendung als photostabile UV-Filter in kosmetischen oder pharmazeutischen Zubereitungen wird in DE-A-199 17 906 beschrieben.

Gemäß DE-A-199 17 906 können die o.g. 2-(4-N,N-Dialkylamino-2-hydroxybenzoyl)benzoesäurealkylester durch direkte Acylierung der entsprechenden aminosubstituierten Phenole mit Phthalsäureanhydrid zu Ketosäuren und anschließende Veresterung hergestellt werden.

Bekannterweise liefert die Umsetzung aminosubstituierter Phenole mit Phthalsäureanhydrid als Nebenprodukte Rhodamine, die zu einer unerwünschten Verfärbung der gebildeten Ketosäuren führen.

Zur Vermeidung/Verringerung der Rhodamin-Bildung beschreibt EP-B-0 511 019 ein Verfahren zur Herstellung einer Ketosäure der allgemeinen Formel 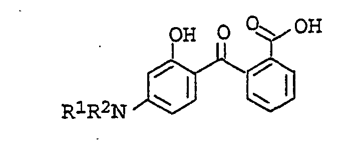 wobei R¹ und R² unabhängig Alkyl mit 1-6 Kohlenstoffatomen oder Cycloalkyl mit 4-8 Kohlenstoffatomen bedeuten, umfassend die Umsetzung eines m-Aminophenols der allgemeinen Formel 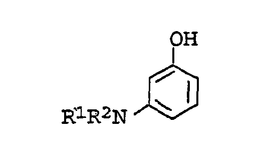 mit Phthalsäureanhydrid in Gegenwart eines organischen Lösungsmittels, wobei das organische Lösungsmittel in einer Menge von 0,5 bis 3 Gew.-Teilen pro 1 Gew.-Teil des m-Aminophenols vorhanden ist, mit der Wirkung, daß die erhaltene Ketosäure in dem Lösungsmittel abgeschieden wird; so daß die Reaktion in Aufschlämmung durchgeführt wird.

EP-A-0 853 079 beinhaltet ebenfalls ein Verfahren zur Herstellung der o.g. Ketosäuren durch Umsetzung eines m-Aminophenols mit Phthalsäure in Gegenwart eines organischen Lösungsmittels, wobei das organische Lösungsmittel in einer Menge von weniger als 0,5 Gew.-Teilen pro 1 Gew.-Teil des m-Aminophenols vorhanden ist.

Auch die nach den oben genannten Verfahren hergestellten 2-(4-N,N-Dialkylamino-2-hydroxybenzoyl)benzoesäurealkylester zeigen häufig noch eine unerwünschte Verfärbung und genügen somit nicht den hohen Qualitätsstandards, die von diesen Verbindungen für die Verwendung als UV-Filter in kosmetischen Zubereitungen gefordert werden.

Es war daher die Aufgabe der vorliegenden Erfindung, ein Verfähren zur Herstellung von 2-(4-N,N-Diethylamino-2-hydroxybenzoyl)benzoesäure-n-hexylester bereitzustellen, das einfach durchführbar ist und zu einem farblosen Produkt mit hoher Reinheit führt.

Diese Aufgabe wurde gelöst durch ein Verfahren zur Herstellung von 2-(4-N,N-Diethylamino-2-hydroxybenzoyl)benzoesäure-n-hexylester der Formel Ia, 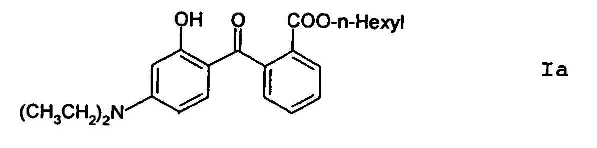 durch
I. Umsetzung von 3-N,N-Diethylaminophenol der Formel IIa mit Phthalsäureanhydrid der Formel III zu 2-(4-N,N-Diethyl-amino-2-hydroxybenzoyl)benzoesäure der Formel IVa und 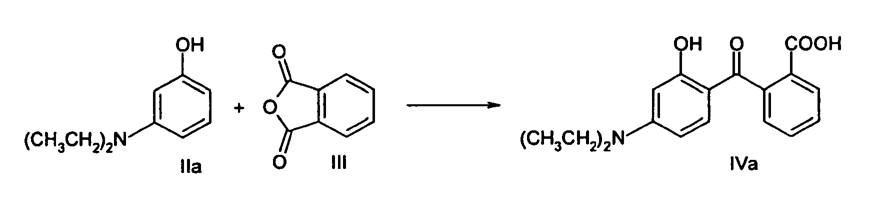
II. anschließende Veresterung der in der I. Stufe gebildeten 2-(4-N,N-Diethylamino-2-hydroxybenzoyl)benzoesäure der Formel IVa mit n-Hexanol in Gegenwart eines sauren Katalysators zum 2-(4-N,N-Diethylamino-2-hydroxybenzoyl)benzoesäure-n-hexylester der Formel Ia, 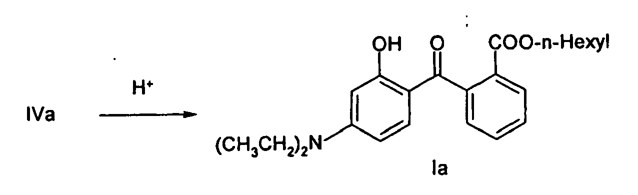
dadurch gekennzeichnet, dass man den gebildeten Ester der Formel Ia kristallisiert und die Kristalle in einer weiteren Verfahrensstufe III durch Behandlung mit einem Adsorbens und durch Destillation aufreinigt, wobei als Adsorbens Aktivkohle oder Kieselgel verwendet wird.

Die Acylierung im Verfahrensschritt I. erfolgt bevorzugt in Gegenwart eines Lösungsmittels. Die hierfür verwendeten Lösungsmittel sind beispielsweise aromatische Kohlenwasserstoffe wie Benzol, Toluol oder Xylol, aliphatische C₈-C₁₂-Kohlenwasserstoffe wie Octan, Isooctan oder Decan, Ether wie Diethylether, Dibutylether oder Tetrahydrofuran sowie chlorierte Kohlenwasserstoffe wie Perchlorethylen oder Chlorbenzol. Besonders bevorzugt verwendete Lösungsmittel sind Toluol und Xylol.

Die Menge an verwendetem Lösungsmittel wird in der Regel so gewählt, dass die gebildete Ketosäure bereits während der Reaktion auskristallisiert.

Es ist auch möglich, für die Acylierung ein Lösungsmittel in Mengen im Bereich von größer 3,5 Gew.-Teilen pro 1 Gew.-Teil des 3-N,N-Dialkylaminophenols durchzuführen, wobei es sich als vorteilhaft erwiesen hat, wenn man in diesem Fall einen Teil des Lösungsmittels während der Reaktion wieder abdestilliert.

Die Reaktionstemperatur, bei der die Acylierung erfolgt, liegt in der Regel im Bereich zwischen 50°C und 150°C, bevorzugt bei der Siedetemperatur des verwendeten Lösungsmittels.

Das Molverhältnis der Reaktanten Phthalsäureanhydrid zu 3-N,N-Dialkylaminophenol liegt in der Regel im Bereich von 0,7 : 1 bis 2 : 1, bevorzugt im Bereich von 1 : 1 bis 1,5 : 1.

Nach Beendigung der Reaktion kann die gebildete Ketosäure [2-(4-N,N-Dialkylamino-2-hydroxybenzoyl)benzoesäure] nach Abkühlung des Reaktionsgemisches auf Temperaturen im Bereich zwischen 0°C und 60°C, bevorzugt zwischen 10°C und 50°C, besonders bevorzugt zwischen 30°C und 50°C abfiltriert, mit dem Lösungsmittel gewaschen und anschließend direkt und ohne Trocknung in die zweite Stufe (Veresterung) eingesetzt werden.

Die Veresterung der in Stufe I gebildeten 2-(4-N,N-Diethyl-amino-2-hydroxybenzoyl)benzoesäure IV erfolgt in an sich bekannter weise (siehe dazu Organikum, VEB Deutscher Verlag der Wissenschaften, Berlin 1986, 16. Auflage, Seite 400-408) mit n-Hexanol in Gegenwart eines sauren Katalysators. Der verwendete Alkohol kann dabei sowohl als Reagenz als auch Lösungsmittel fungieren. Zur Erhöhung der Ausbeute ist es vorteilhaft, wenn man das bei der Veresterung entstandene Reaktionswasser azeotrop abdestilliert.

Als saure Katalysatoren können z.B. HC1, H₂SO₄, HNO₃, Phosphorsäure, Sulfonsäuren wie Benzolsulfonsäure, p-Toluolsulfonsäure, Methansulfonsäure oder Mischungen dieser Säuren, aber auch sulfonsäuregruppen-haltige Ionenaustauscher, wie z.B. Lewatit® S100 (Fa. Bayer) verwendet werden. Bevorzugte saure Katalysatoren sind HCl, H₂SO₄ , Methansulfonsäure und p-Toluolsulfonsäure.

Eine besonders bevorzugte Ausführungsform des erfindungsgemäßen Verfahrens ist dadurch gekennzeichnet, dass die Veresterung in der Verfahrensstufe II in Gegenwart von Schwefelsäure als Katalysator erfolgt.

Nach Beendigung der Veresterung wird das Reaktionsgemisch neutralisiert und der Ester nach Abtrennung der wäßrigen Phase isoliert.

Bei den im Verfahrensschritt III verwendeten Adsorbentien handelt es sich generell um feste Stoffe, die aufgrund ihrer großen Oberfläche befähigt sind, Verunreinigungen aus flüssigen Mischungen an ihrer Grenzfläche selektiv zu adsorbieren. Bevorzugt sind Aktivkohlen und Kieselgele.

Die als Adsorbentien in Frage kommenden Kieselgele sind u.a. beschrieben im CD Römpp Chemie Lexikon - Version 1.0, Stichwort: Kieselgele, Stuttgart/New York: Georg Thieme Verlag 1995 und der darin zitierten Literatur. Bevorzugte Kieselgele sind Kieselgel 60 der Fa. Merck, Darmstadt und Silicagel 123 der Fa. Grace.

Eine bevorzugte Ausführungsform des Verfahrens ist die Aufreinigung des gebildeten Esters der Formel Ia durch Behandlung mit Aktivkohle. Dabei kann die Aktivkohle in Pulverform, Granulatform oder als zylindrisch geformte Teilchen eingesetzt werden. Vorteilhafterweise wird dabei die Aktivkohle in Granulatform (Korn-Aktivkohle) in Fest- oder Fließbettfiltern eingesetzt. Beispiele für bevorzugt verwendete Kohlen sind die Aktivkohlen CPG^{®} LF, CAL^{®} und APC^{®} der Fa. Chemviron Carbon. Nähere Einzelheiten über Eigenschaften und Qualitäten der verwendeten Aktivkohlen finden sich in Ullmann's Encyclopedia, Sixth Edition, 2000 Electronic Release, Chapter 5.

Im Falle der Kieselgele ist es ebenfalls von Vorteil, wenn diese Adsorbentien als Festbett eingesetzt werden.

Die Menge an verwendetem Adsorbens liegt im Bereich von 0,001 bis 0,2 g, bevorzugt 0,05 bis 0,1 g, bezogen auf jeweils 1 g des aufzureinigenden Esters I.

Das erfindungsgemäße Verfahren zeichnet sich außerdem dadurch aus, dass der gebildete 2-(4-N,N-Diethylamino-2-hydroxybenzoyl)benzoesäure-n-hexylester der Formel Ia weniger als 10 ppm, bevorzugt weniger als 5 ppm, besonders bevorzugt weniger als 1 ppm Rhodamin enthält.

Eine besonders bevorzugte Ausführungsform des Verfahrens unter Verwendung von Aktivkohle ist dadurch gekennzeichnet, dass man in der Verfahrensstufe III
a. den Ester in einem unpolaren Lösungsmittel bei einer Temperatur im Bereich von 10°C bis 100°C, bevorzugt im Bereich von 20°C bis 80°C, besonders bevorzugt im Bereich von 25°C bis 50°C löst,
b. diese Lösung über ein Korn-Aktivkohlebett bei einer Temperatur m Bereich von 10°C bis 100°C, bevorzugt im Bereich von 20°C bis 80°C, besonders bevorzugt im Bereich von 25°C bis 50°C leitet,
c. den Ester nach Passieren des Korn-Aktivkohlebetts destillativ vom Lösungsmittel abtrennt.

Als unpolare Lösungsmittel bezeichnet man im Rahmen der Erfindung solche Lösungsmittel mit niedriger Dielektrizitätskonstanten (ε < 15) und kleinem Dipolmoment (µ = 0 bis 2). Beispiele sind u.a. Petrolether, Ligroin, n-Hexan, Cyclohexan, Heptan, Din-butylether, Xylol, Toluol und Benzol. Als bevorzugtes Lösungsmittel im Verfahrensschritt IIIa verwendet man Toluol, Hexan oder Cyclohexan, besonders bevorzugt Toluol oder Cyclohexan, ganz besonders bevorzugt Toluol.

Es ist ferner möglich, dass man in der Verfahrensstufe III
a. den Ester in einem Alkohol, insbesondere in Hexanol bei einer Temperatur im Bereich von 10°C bis 100°C, bevorzugt im Bereich von 20°C bis 80°C, besonders bevorzugt im Bereich von 25°C bis 50°C löst,
b. diese Lösung über ein Kieselgelbett bei einer Temperatur m Bereich von 10°C bis 100°C, bevorzugt im Bereich von 20°C bis 80°C, besonders bevorzugt im Bereich von 25°C bis 50°C leitet,
c. den Ester nach Passieren des Kieselgelbetts destillativ vom Alkohol abtrennt.

Eine ganz besonders bevorzugte Ausführungsform des Verfahrens betrifft die Herstellung des 2-(4-N,N-Diethylamino-2-hydroxybenzoyl)benzoesäure-n-hexylesters der Formel Ia 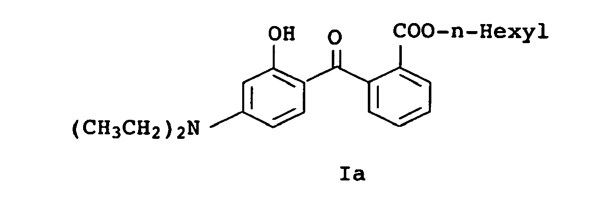 durch
I. Umsetzung von 3-N,N-Diethylaminophenol der Formel IIa mit Phthalsäureanhydrid der Formel III zu 2-(4-N,N-Diethyl-amino-2-hydroxybenzoyl)benzoesäure der Formel IVa, 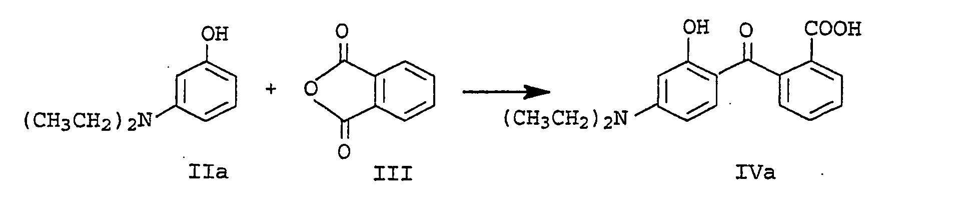
II. Veresterung der in der I. Stufe gebildeten 2-(4-N,N-Diethylamino-2-hydroxybenzoyl)benzoesäure der Formel IVa in Hexanol in Gegenwart von Schwefelsäure zum 2-(4-N,N-Diethylamino-2-hydroxybenzoyl)benzoesäure-n-hexylester der Formel Ia 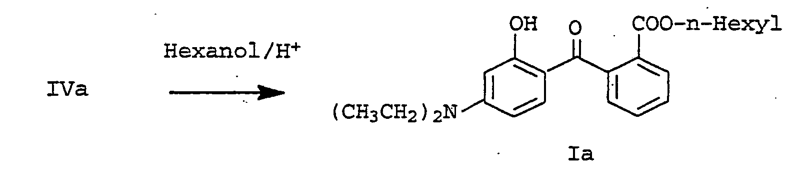 und Isolierung des n-Hexylesters Ia in kristalliner Form,
III.
   a. Lösen des n-Hexylesters Ia in Toluol oder Hexanol bei einer Temperatur im Bereich von 25°C bis 50°C,
   b. Dosierung dieser Lösung über ein Korn-Aktivkohlebett oder ein Kieselgelbett bei einer Temperatur im Bereich von 25°C bis 50°C und,
   c. Isolierung des n-Hexylesters durch destillative Abtrennung des Toluols und/oder Hexanols.

Die destillative Aufreinigung erfolgt in der Regel so, daß zunächst das Lösungsmittel beispielsweise über einen Fallfilm- oder Dünnschichtverdampfer unter Vakkuum abgetrennt wird und der das Wertprodukt-enthaltende Rückstand anschließend über eine Kolonne destilliert wird.

Der so erhaltene farblose Ester kann nach der Destillation bevorzugt als Schmelze verpackt werden.

Die folgenden Beispiele sollen das erfindungsgemäße Verfahren näher erläutern.

### Beispiel 1

### Herstellung von 2-(4-N,N-Diethylamino-2-hydroxybenzoyl)benzoesäure

In einem 500 mL Vierhalskolben mit Teflonrührer, Thermometer und Rückflusskühler wurden unter Stickstoffatmosphäre 99 g (0,60 mol) 3-Diethylaminophenol 93,2 g (0,63 mol) Phthalsäureanhydrid und 460 ml Toluol eingefüllt und auf Rückflusstemperatur erwärmt. Nach 2 Stunden Reaktionszeit wurden innerhalb von 30 Minuten insgesamt 300 g Toluol abdestilliert und anschließend 3 Stunden unter Rückfluss gerührt. Der Ansatz wurde auf Raumtemperatur abgekühlt und abgesaugt. Der Filterkuchen wurde nacheinander mit 90 ml Toluol und zweimal mit je 90 ml Hexanol gewaschen. Die hexanolfeuchte Säure konnte direkt in der zweiten Stufe eingesetzt werden. Ausbeute: 169 g (90 %) rosa gefärbte 2-(Diethylamino-2-hydroxybenzoyl)benzoesäure.

### Beispiel 2

### Herstellung von 2-(4-N,N-Diethylamino-2-hydroxybenzoyl)benzoesäure-n-hexylester

In einem 1 1 Planschliffkolben mit Ankerrührer, Stickstoffeinleitung, Thermoelement und Wasserauskreiser wurden 313 g (1,0 mol) hexanolfeuchte 2-(4-Diethylamino-2-hydroxybenzoyl)-benzoesäure (ber. 100%) und 750 ml n-Hexanol vorgelegt, mit 53 g (519 mmol) 96%iger Schwefelsäure versetzt und auf eine Innentemperatur von 105-110°C erwärmt. Im Anschluss an die Aufwärmphase wurde das gebildete Reaktionswasser innerhalb von 6-8 h bei einer Innentemperatur von 105-110°C und einem Druck von ca, 200 mbar azeotrop abdestilliert. Nach dem Abkühlen auf ca. 70°C wurde der Ansatz mit 830 ml Wasser versetzt und bei einer Temperatur von 52-58°C mit 25 %iger NaOH-Lösung neutralisiert. Die wässrige Phase wurde abgetrennt und die organische Phase mit 500 ml Wasser extrahiert (Temperatur: 52-58°C). Die wässrige Phase wurde abgetrennt, die organische Phase auf 20°C abgekühlt und die Kristallisation des Esters abgewartet. Im Anschluss an eine 1 stündige Haltephase zur Reifung des in-situ erzeugten Impfmaterials wurde mit 5 K/h auf 0 bis 5°C abgekühlt, 2 h bei dieser Temperatur nachgerührt und abgesaugt. Der Filterkuchen wurde zweimal mit je 85 ml kaltem Hexanol gewaschen. Das hexanolfeuchte, rosa gefärbte Rohprodukt (407 g) wurde gut trockengesaugt und durch Adsorption an Aktivkohle gereinigt.

### Beispiel 3

### Aufreinigung von 2-(4-N,N-Diethylamino-2-hydroxybenzoyl)benzoesäure-n-hexylester mittels Korn-Aktivkohle

200 g feuchter 2-(4-N,N-Diethylamino-2-hydroxybenzoyl)benzoesäure-n-hexylester, erhältlich nach Beispiel 2, wurden in 400 ml Toluol gelöst und bei 25°C über eine mit Aktivkohle CPG^{®} LF der Fa. Chemviron Carbon gefüllte Säule gegeben. Das farblose Eluat wurde mittels Fallfilmverdampfer eingeengt und der ölige Rückstand in einer Destillationskolonne bei einem Druck von 100 mbar im Gegenstrom mit heißem Stickstoff auf < 10 ppm Toluol abgereichert. Anschließend wurde das wertprodukt als Schmelze abgefüllt.

## Patentansprüche

1. Verfahren zur Herstellung von 2-(4-N,N-Diethylamino-2-hydroxybenzoyl)benzoesäure-n-hexylester der Formel Ia 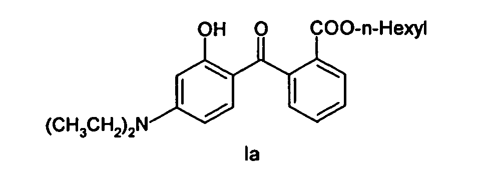 durch
I. Umsetzung von 3-N,N-Diethylaminophenol der Formel IIa mit Phthalsäureanhydrid der Formel III zu 2-(4-N,N-Diethylamino-2-hydroxybenzoyl)benzoesäure der Formel IVa und 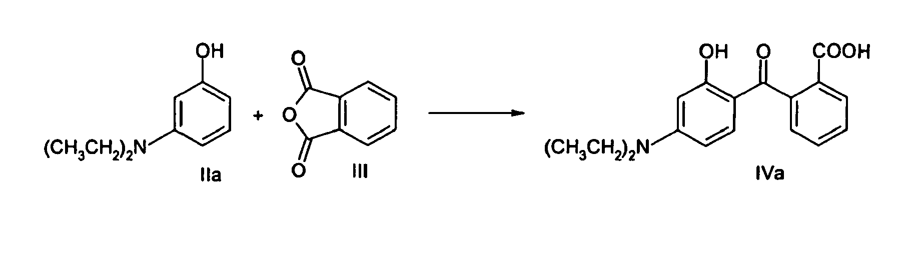
II. anschließende Veresterung der in der I. Stufe gebildeten 2-(4-N,N-Diethylamino-2-hydroxybenzoyl)benzoesäure der Formel IVa mit n-Hexanol in Gegenwart eines sauren Katalysators zum 2-(4-N,N-Diethylamino-2-hydroxybenzoyl)benzoesäure-n-hexylester der Formel Ia, 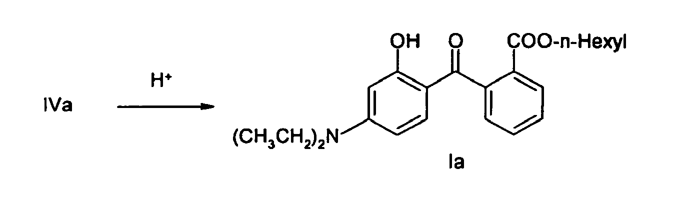
**dadurch gekennzeichnet, dass** man den gebildeten Ester der Formel Ia kristallisiert und die Kristalle in einer weiteren Verfahrensstufe III durch Behandlung mit einem Adsorbens und durch Destillation aufreinigt, wobei als Adsorbens Aktivkohle oder Kieselgel verwendet wird.

2. Verfahren nach Anspruch 1, **dadurch gekennzeichnet, dass** die Veresterung in der Verfahrensstufe II in Gegenwart von Schwefelsäure als Katalysator erfolgt.

3. Verfahren nach einem der Ansprüche 1 oder 2, **dadurch gekennzeichnet, dass** der gebildete 2-(4-N,N-Diethylamino-2-hydroxybenzoyl)benzoesäure-n-hexylester der Formel Ia weniger als 10 ppm Rhodamin enthält.

4. Verfahren nach einem der Ansprüche 1 bis 3, **dadurch gekennzeichnet, dass** in der Verfahrensstufe III der Ester durch Behandlung mit Aktivkohle und anschließender Destillation aufgereinigt wird.

5. Verfahren nach Anspruch 4, **dadurch gekennzeichnet, dass** man in der Verfahrensstufe III
a. den Ester in einem unpolaren Lösungsmittel bei einer Temperatur im Bereich von 10°C bis 100°C löst,
b. diese Lösung über ein Korn-Aktivkohlebett bei einer Temperatur im Bereich von 20°C bis 100°C leitet,
c. den Ester nach Passieren des Korn-Aktivkohlebetts destillativ vom Lösungsmittel abtrennt.

6. Verfahren nach Anspruch 5, **dadurch gekennzeichnet, dass** man als Lösungsmittel im Verfahrensschritt IIIa Cyclohexan oder Toluol verwendet.

7. Verfahren zur Herstellung von 2-(4-N,N-Diethylamino-2-hydroxybenzoyl)benzoesäure-n-hexylylester der Formel Ia 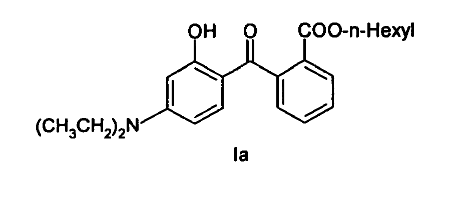 durch
I. Umsetzung von 3-N,N-Diethylaminophenol der Formel IIa mit Phthalsäureanhydrid der Formel III zu 2-(4-N,N-Diethylamino-2-hydroxybenzoyl)benzoesäure der Formel IVa, 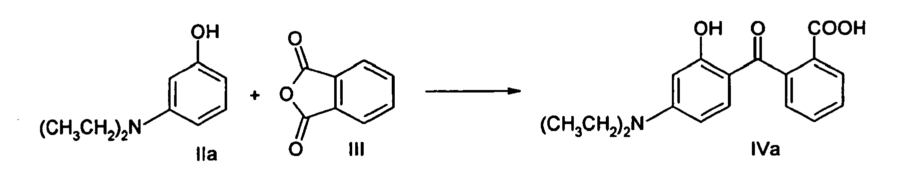
II. Veresterung der in der I. Stufe gebildeten 2-(4-N,N-Diethylamino-2-hydroxybenzoyl)benzoesäure der Formel IVa in Hexanol in Gegenwart von Schwefelsäure zum 2-(4-N,N-Diethylamino-2-hydroxybenzoyl)benzoesäure-n-hexylester der Formel Ia 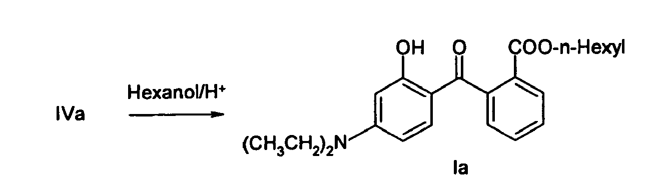 und Isolierung des n-Hexylesters Ia in kristalliner Form,
III.
a. Lösen des n-Hexylesters Ia in Toluol oder Hexanol bei einer Temperatur im Bereich von 25°C bis 50°C,
b. Dosierung dieser Lösung über ein Korn-Aktivkohlebett oder ein Kieselgelbett bei einer Temperatur im Bereich von 25°C bis 50°C und
c. anschließende Isolierung des n-Hexylesters durch destillative Abtrennung des Toluols und/oder Hexanols.

## Claims

1. A process for the preparation of n-hexyl 2-(4-N,N-diethylamino-2-hydroxybenzoyl)benzoate of the formula Ia, 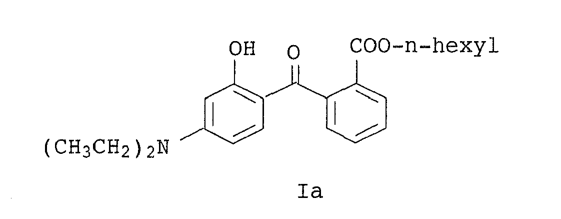 by
I. reaction of 3-N,N-diethylaminophenol of the formula IIa, with phthalic anhydride of the formula III to give 2-(4-N,N-diethylamino-2-hydroxybenzoyl)benzoic acid of the formula IVa and 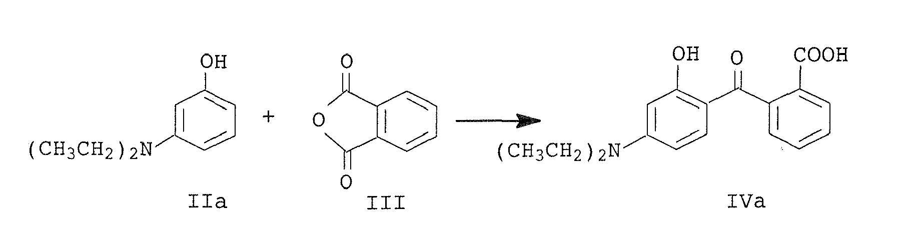
II. subsequent esterification of the 2-(4-N,N-diethylamino-2-hydroxybenzoyl)benzoic acid of the formula IVa formed in stage I with n-hexanol in the presence of an acidic catalyst to give n-hexyl 2-(4-N,N-diethylamino-2-hydroxybenzoyl)benzoate of the formula Ia, 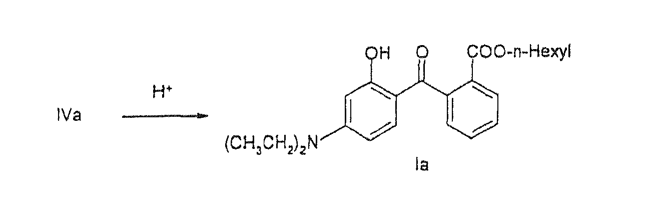
which comprises crystallizing the ester of the formula Ia formed and purifying the crystals in a further process stage III by treatment with an adsorbent and by distillation, the adsorbent used being activated carbon or silica gel.

2. The process according to claim 1, wherein the esterification in process stage II is carried out in the presence of sulfuric acid as catalyst.

3. The process according to either of claims 1 and 2, wherein the n-hexyl 2-(4-N,N-diethylamino-2-hydroxybenzoyl)benzoate of the formula Ia formed comprises less than 10 ppm of rhodamine.

4. The process according to one of claims 1 to 3, wherein, in process stage III, the ester is purified by treatment with activated carbon and subsequent distillation.

5. The process according to claim 4, wherein, in process stage III
a. the ester is dissolved in a nonpolar solvent at a temperature in the range from 10°C to 100°C,
b. this solution is passed over a granular activated carbon bed at a temperature in the range from 20°C to 100°C,
c. the ester, after passing through the granular activated carbon bed, is separated off from the solvent by distillation.

6. The process according to claim 5, wherein the solvent used in process step IIIa is cyclohexane or toluene.

7. A process for the preparation of n-hexyl 2-(4-N,N-diethylamino-2-hydroxybenzoyl)benzoate of the formula Ia 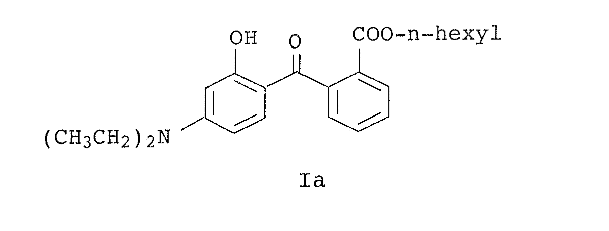 by
I. reaction of 3-N,N-diethylaminophenol of the formula IIa with phthalic anhydride of the formula III to give 2-(4-N,N-diethylamino-2-hydroxybenzoyl)benzoic acid of the formula IVa, 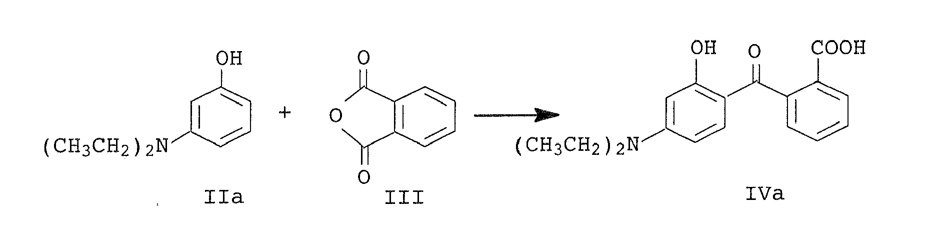
II. esterification of the 2-(4-N,N-diethylamino-2-hydroxybenzoyl)benzoic acid of the formula IVa formed in stage I in hexanol in the presence of sulfuric acid to give n-hexyl 2-(4-N,N-diethylamino-2-hydroxybenzoyl)benzoate of the formula Ia 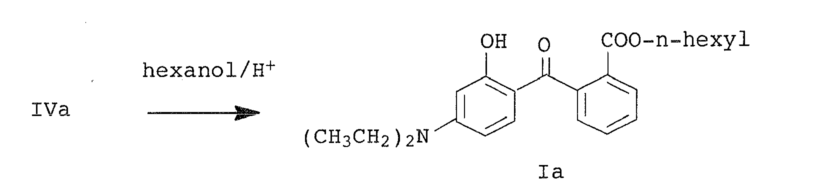 and isolation of the n-hexyl ester Ia in crystalline form,
III.
a. dissolution of the n-hexyl ester Ia in toluene or hexanol at a temperature in the range from 25°C to 50°C,
b. metering of this solution over a granular activated carbon bed or a silica gel bed at a temperature in the range from 25°C to 50°C and
c. subsequent isolation of the n-hexyl ester by separating off the toluene and/or hexanol by distillation.

## Revendications

1. Procédé de préparation n-hexylester d'acide 2-(4-N,N-diéthylamino-2-hydroxybenzoyl)benzoïque de formule la 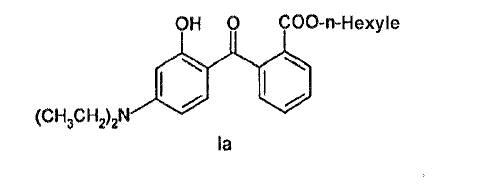 par
I, réaction de 3-N,N-diéthylaminophénol de formule IIa avec de l'anhydride d'acide phtalique de formule III en acide 2-(4-N,N-diéthylamino-2-hydroxybenzoyl)benzoïque de formule IVa et 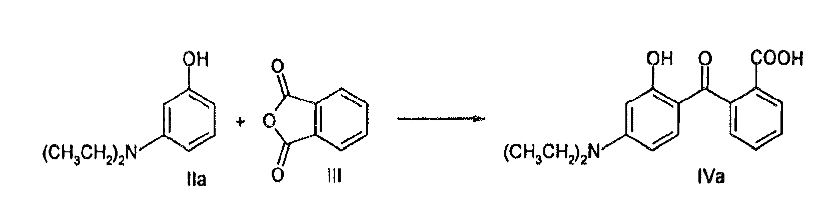
II. estérification subséquente de l'acide 2-(4-N,N-diéthylamino-2-hydroxybenzoyl)benzoïque formé à l'étape I de formule IVa avec du n-hexanol en présence d'un catalyseur acide pour obtenir du n-hexylester d'acide 2-(4-N,N-diéthylamino-2-hydroxybenzoyl)benzoïque de formule Ia, 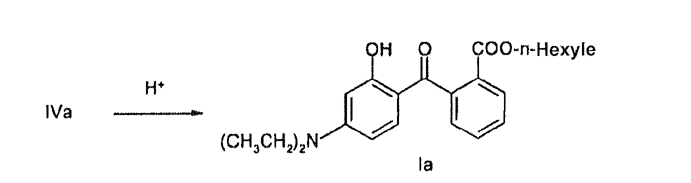
**caractérisé en ce qu'**on cristallise l'ester formé de formule la et qu'on purifie les cristaux lors d'une autre étape de procédé III par traitement avec un adsorbant et par distillation, au cours de laquelle on utilise comme adsorbant du charbon actif ou du gel de silice.

2. Procédé selon la revendication 1, **caractérisé en ce que** l'estérification dans l'étape de procédé II s'effectue en présence d'acide sulfurique comme catalyseur.

3. Procédé selon l'une des revendications 1 ou 2, **caractérisé en ce que** le n-hexylester d'acide 2-(4-N,N-diéthylamino-2-hydroxybenzoyl)benzoïque formé de formule la contient moins de 10 ppm de rhodamine.

4. Procédé selon l'une des revendications 1 à 3, **caractérisé en ce que** lors de l'étape de procédé III l'ester est purifié par traitement avec du charbon actif et distillation subséquente.

5. Procédé selon la revendication 4, **caractérisé en ce que**, lors de l'étape de procédé III :
a. on dissout l'ester dans un solvant non polaire et à une température dans l'intervalle compris entre 10°C et 100°C,
b. on conduit cette solution par un lit de charbon actif en granules à une température dans l'intervalle compris entre 20°C et 100°C,
c. on sépare du solvant l'ester après passage du lit de charbon actif en granules par distillation.

6. Procédé selon la revendication 5, **caractérisé en ce qu'**on utilise comme solvant lors de l'étape de procédé IIIa, du cyclohexane ou du toluène.

7. Procédé de préparation de n-hexylester d'acide 2-(4-N,N-diéthylamino-2-hydroxybenzoyl)benzoïque de formule Ia 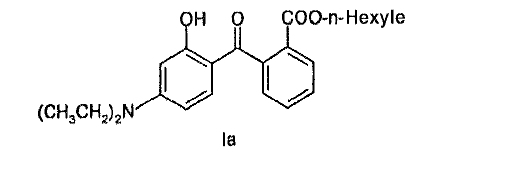 par
I. réaction de 3-N,N-diéthylaminophénol de formule IIa avec de l'anhydride d'acide phtalique de formule III en acide 2-(4-N,N-diéthylamino-2-hydroxybenzoyl)benzoïque de formule IVa, 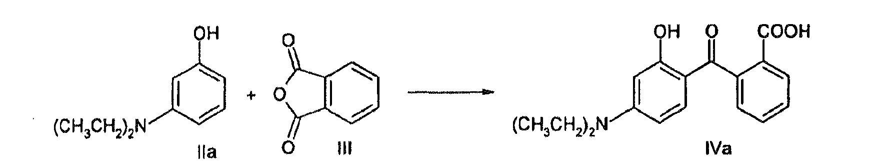
II. estérification de l'acide 2-(4-N,N-diéthylamino-2-hydroxybenzoyl)benzoïque formé à l'étape I de formule IVa dans de l'hexanol en présence d'acide sulfurique pour obtenir du n-hexylester d'acide 2-(4-N,N-diéthylamino-2-hydroxybenzoyl)benzoïque de formule Ia, 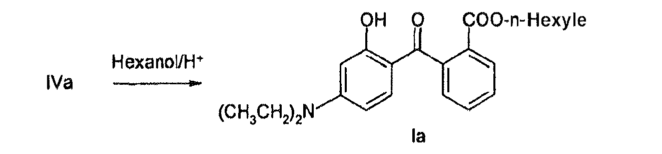 et isolation du n-hexylester la sous forme cristalline,
III.
a. dissolution du n-hexylester Ia dans du toluène ou de l'hexanol à une température dans l'intervalle compris entre 25°C et 50°C,
b. dosage de cette solution par un lit de charbon actif en granules ou un lit de gel de silice à une température dans l'intervalle compris entre 25°C et 50°C, et
c. isolation subséquente du n-hexylester par séparation par distillation du toluène et/ou de l'hexanol.
